# EUROPÄISCHE PATENTSCHRIFT

(11) **EP 0 829 723 B1**
(45) Veröffentlichungstag und Bekanntmachung des Hinweises auf die Patenterteilung: **15.03.2006**
(21) Anmeldenummer: 97115824.1
(22) Anmeldetag: 11.09.1997
(51) Int. Cl.: G01N 33/546, G01N 33/551, G01N 33/554, G01N 33/543

(54) **Homogene Nachweisverfahren zur Bestimmung von Subpopulationen eines Analyten**
Homogeneous detection method for the determination of sub-populations of an analyte
Méthode de détection homogène pour la détermination de sous-populations d'un analyte

(30) Priorität: 13.09.1996 DE 19637418
(43) Veröffentlichungstag der Anmeldung: 18.03.1998
(73) Patentinhaber: Roche Diagnostics GmbH, 68305 Mannheim-Waldhof (DE)
(72) Erfinder: Hösel, Wolfgang, Dr., 82327 Tutzing (DE); Mutter, Wolfgang, Dr., 82347 Bernried (DE)
(74) Vertreter: Weiss, Wolfgang

(56) Entgegenhaltungen:
- EP-A- 0 074 520
- EP-A- 0 166 623
- EP-A- 0 260 079
- EP-A- 0 323 909
- EP-A- 0 337 410
- EP-A- 0 516 529
- WO-A-84/04396
- DE-A- 4 309 393

## Beschreibung

Die vorliegende Erfindung betrifft ein Verfahren zur Bestimmung eines Analyten in einer Probeflüssigkeit durch Bindung an einen ersten mit dem Analyten spezifisch bindefähigen Rezeptor, der auf einem in der Probeflüssigkeit befindlichen teilchenförmigen Trägermaterial immobilisiert ist, und durch Agglutination mit einem zweiten Analyt-spezifischen Rezeptor, sowie eine neue Reagenzienkombination zur Durchführung eines derartigen Verfahrens. Insbesondere ist dieses Verfahren zur qualitativen oder/und quantitativen Bestimmung von Subpopulationen eines Analyten geeignet.

Immunologische Nachweisverfahren nach dem Agglutinationsprinzip sind seit langem bekannt. Dabei wird ein zu bestimmender Analyt mit einem gegen den Analyten gerichteten Rezeptor oder Bindepartner unter Bedingungen, bei denen eine Agglutinierung der Reaktionspartner erfolgt, vermischt. Die durch die immunologische Reaktion hervorgerufene Agglutinierung führt zu Teilchenaggregaten, die sich im Streulichtverhalten von den Ausgangssubstanzen unterscheiden, wodurch eine Bestimmung der Analytenkonzentration ermöglicht wird.

Auch die Verwendung von mehreren gegen den zu bestimmenden Analyten gerichteten Rezeptoren in einem immunologischen Nachweisverfahren ist bekannt. So beschreibt DE-OS 36 33 497 ein immunometrisches Bestimmungsverfahren für eine mindestens zwei Antikörperbindungsstellen aufweisende antigene Substanz, bei dem man eine diese Substanz enthaltende Flüssigkeitsprobe mit einem immobilisierten, unmarkierten ersten spezifischen Antikörper und einem zweiten spezifischen markierten Antikörper sequentiell oder in einem Schritt inkubiert, wobei sich ein nachweisbarer festphasengebundener ternärer Komplex aus der zu bestimmenden Substanz und den beiden Antikörpern bildet. In einer ersten Messung wird die Antigenkonzentration über den gesamten Meßbereich ermittelt und danach wird die Probe einer zweiten Messung unterzogen.

In US-A-4,595,661 werden Sandwich- und nephelometrische Immuntests beschrieben, die zur Verringerung des Hook-Effekts neben den normalerweise in einem Immuntest vorhandenen hochspezifischen Antikörpern zusätzlich mindestens einen weiteren Antikörper enthalten, der eine geringe Affinität für den Analyten besitzt.

EP-A-0 572 845 offenbart ein Verfahren zur immunchemischen Bestimmung eines Analyten in einer Probe mittels eines ersten immobilisierten spezifischen Bindepartners und eines zweiten spezifischen Bindungspartners, der direkt oder indirekt eine Markierung trägt, wobei zusätzlich ein Bindefaktor zugegeben wird, welcher mehr als eine bindungsfähige Stelle für den nachzuweisenden Analyten aufweist und keine Affinität zu den immobilisierten spezifischen Bindungspartner aufweist und der nicht markiert ist.

DE-OS 43 09 393.0 offenbart ein Verfahren zur Bestimmung eines Analyten durch Verwendung eines ersten Analyt-spezifischen Rezeptors, der auf einem teilchenförmigen Trägermaterial immobilisiert ist, und eines zweiten löslichen Analyt-spezifischen Rezeptors, der mindestens 2 Bindungsstellen für den Analyten besitzt.

Auch in den japanischen Offenlegungsschriften 4-344465, 1-301165 und 59-92353 wird die Verwendung mehrerer Analytspezifischer Rezeptoren in einem immunologischen Nachweisverfahren offenbart.

Keines der oben beschriebenen Verfahren eignet sich jedoch als homogenes Nachweisverfahren zur Bestimmung von Subpopulationen eines Analyten, z.B. zur quantitativen Bestimmung des Vorhandenseins bestimmter Kohlenhydratstrukturen in Glykoproteinen. Die bekannten homogenen Immunoassays dienen nur jeweils der Bestimmung der Gesamtmenge eines Analyten, wobei eventuell zu bestimmende Subpopulationen des Analyten in einem Probenvorbereitungsschritt isoliert und dann erst in dem eigentlichen Test bestimmt werden. Solche Verfahren sind jedoch aufwendig und können darüberhinaus zu Fehlern, z.B. aufgrund von Verlusten bei der Isolation der zu bestimmenden Subpopulationen, führen. Um diese Nachteile zu vermeiden, mußten bisher heterogene Immunoassays mit bound/free-Trennungen durchgeführt werden, die aber wiederum einen wesentlich größeren Aufwand erfordern als homogene Immunoassays.

Die der vorliegenden Erfindung zugrundeliegende Aufgabe bestand darin, ein neues Verfahren zur Bestimmung von Subpopulationen eines Analyten bereitzustellen, bei dem die Nachteile des Standes der Technik mindestens teilweise beseitigt sind.

Diese Aufgabe wird gelöst durch ein Verfahren zur Bestimmung eines Analyten in einer Probeflüssigkeit, welches dadurch gekennzeichnet ist, daß man die Probeflüssigkeit mit (a) einem einzigen ersten Rezeptor, der an ein erstes, nur einmal auf dem Analyten vorhandenes Epitop bindet und auf einem teilchenförmigen Trägermaterial immobilisiert ist, und (b) einem zweiten Rezeptor, der an ein zweites Epitop auf dem Analyten bindet und mindestens zwei Bindungsstellen für das zweite Epitop aufweist, inkubiert, wobei das erste Epitop verschieden vom zweiten Epitop ist, und den Analyten über die Bindung an beide Rezeptoren bestimmt.

Die Erfindung basiert auf dem Konzept, daß ein einziger erster Rezeptor, der gegen ein nur einmal auf dem zu bestimmenden Analyten, z.B. einem Makromolekül, vorhandenen Epitop gerichtet ist, an die Oberfläche des teilchenförmigen Trägermaterials gebunden wird. Bei Zusammengeben des immobilisierten ersten Rezeptors mit dem Analyten bindet dieser an das teilchenförmige Trägermaterial. Es findet jedoch keine Vernetzung statt, da das vom ersten Rezeptor erkannte Epitop jeweils nur einmal auf dem Analyten vorhanden ist. Bei Zugabe des zweiten Rezeptors, der eine andere, gegebenenfalls auch mehrmals vorhandene spezifische Struktur auf dem Analyten erkennt und der mindestens zwei Bindungsstellen für diese Struktur besitzt, findet eine Vernetzung der Partikel des Trägermaterials über diesen zweiten Rezeptor statt. Falls ein zweiter Rezeptor gewählt wird, der eine nur in einer Subpopulation des zu bestimmenden Analyten vorhandene Struktur erkennt, findet eine Vernetzung nur bei demjenigen Anteil des Analyten statt, der die spezifisch zu erkennende Struktur besitzt. Das Ausmaß und die Schnelligkeit der Vernetzung hängen von der Konzentration dieser Analytensubpopulation ab. Die Bestimmung dieser Konzentrationen kann beispielsweise photometrisch durch Anstieg der Absorption bei einer geeigneten Wellenlänge gemessen werden. Auf diese Weise können Subpopulationen eines Analyten ohne die Notwendigkeit einer bound/free-Trennung oder eines Isolierungsschritts sehr einfach nachgewiesen werden.

Die Reihenfolge der Zugabe der Reaktionspartner ist unkritisch, d.h. die Probe kann zuerst mit dem ersten immobilisierten Rezeptor vermischt und dann der zweite Rezeptor zugegeben werden, oder es können beide Rezeptoren simultan zur Probe gegeben werden.

Beim erfindungsgemäßen Verfahren werden zwei Analytspezifische Rezeptoren verwendet, die jeweils unterschiedliche Epitope, d.h. Oberflächenstrukturen, auf dem Analyten erkennen und an diese vorzugsweise mit hoher Affinität binden. Als Rezeptoren für das erfindungsgemäße Verfahren kommen alle bindefähigen Substanzen in Betracht, die unter Testbedingungen eine ausreichend starke Bindung an den Analyten aufweisen, um eine Bestimmung zu ermöglichen.

Der erste Rezeptor ist an ein teilchenförmiges Trägermaterial immobilisiert. Beispiele für geeignete teilchenförmige Trägermaterialien sind Latexpartikel, anorganische Materialien wie Metall-, Metalloxid- oder Kohlenstoffpartikel, Liposomen oder Zellen wie Bakterienzellen. Die Immobilisierung von Rezeptoren an solche Trägermaterialien und die Verwendung der resultierenden Konjugate in Agglutinationstests ist aus dem Stand der Technik bekannt. Besonders bevorzugte teilchenförmige Trägermaterialien sind Latexpartikel.

Als erster Rezeptor geeignet sind alle bindefähigen Substanzen, die mit einem singulär auf einem Analyten vorhandenen Epitop reagieren können, z.B. Antikörper, Fragmente-und Derivate davon, Lectine, Aptamere und Membranrezeptoren. Konkrete bevorzugte Beispiele für den ersten immobilisierten Rezeptor sind monoklonale Antikörper, monospezifische polyklonale Antikörper oder/und Fragmente derartiger Antikörper. Die Herstellung solcher Antikörper kann beispielsweise durch Verwendung von Immunogenen erfolgen, die eine vorbestimmte, nur einmal auf der Analytoberfläche vorhandene Struktur enthalten, z.B. eine Peptidsequenz. Geeignete Antikörper sind z.B. monoklonale Antikörper gegen Speichelamylase (US-A-4,939,082), Creatinkinase MB (WO94/25617), Follikel-stimulierendes Hormon (EP-86 102 589.8), Luteinisierendes Hormon (US-A-5,248,593) sowie Humanchoriogonadotropin (Boehringer Mannheim, Kat.Nr. 1200 933). Weitere bevorzugte Beispiele für den ersten Rezeptor sind Lectine, die eine nur einmal auf dem Analyten vorhandene Kohlenhydratstruktur erkennen.

Als zweiter Rezeptor, der zur Agglutination dient, kommen alle bindefähigen Substanzen (siehe oben) in Frage, die mindestens zwei Bindungsstellen für ein zweites, auf dem Analyten vorhandenes Epitop besitzen, wobei dieses Epitop vorzugsweise nur auf einer Teilmenge bzw. Subpopulation des Analyten vorkommt. Substanzen, die nur eine einzige Bindungsstelle besitzen, z.B. einzelkettige Antikörper, Aptamere, Membranrezeptoren, können durch Di- oder Polymerisierung in geeignete zweite Rezeptoren überführt werden. Konkrete Beispiele für zweite Rezeptoren sind unter anderem monoklonale Antikörper, die gegen Phosphotyrosin, Phosphoserin und Phosphothreonin gerichtet sind, zur Bestimmung von phosphorylierten Teilmengen bestimmter Proteine; das Lectin Concanavalin A oder monoklonale Antikörper mit entsprechender Spezifität zur Bestimmung von nicht enzymatisch glykierten Proteinen, z.B. Serumalbumin, Hämoglobin; die in den Anwendungsbeispielen beschriebenen Lectine RCA 120 zur Bestimmung von Asialo-Glykoprcteinen sowie SNA zur Bestimmung von sialylierten Glykoproteinen. Analog können zahlreiche weitere Lectine entsprechend ihrer Spezifität zum Nachweis von Glykoproteinen anderer Struktur eingesetzt werden.

Der zweite Rezeptor liegt vorzugsweise in löslicher und nichtimmobilisierter Form vor und ist spezifisch für eine Subpopulation des Analyten, d.h. der zweite Rezeptor erkennt nur eine bestimmte Form des Analyten, der über die Bindung mit dem ersten Rezeptor an das teilchenförmige Trägermaterial immobilisiert ist, während andere Formen des immobilisierten Analyten nicht erkannt werden.

Wenn man als Rezeptorkombination einen Antikörper und ein Lectin verwendet, kann es in manchen Fällen nützlich sein, die auf dem Antikörper vorhandenen Kohlenhydrate zu modifizieren, damit ihre Bindungsfähigkeit für Lectine zerstört wird. Dies kann beispielsweise durch Oxidation durch Perjodat und anschließende Inaktivierung der bei der Oxidation entstandenen Aldehydgruppen durch Behandlung mit Ethanolamin erfolgen.

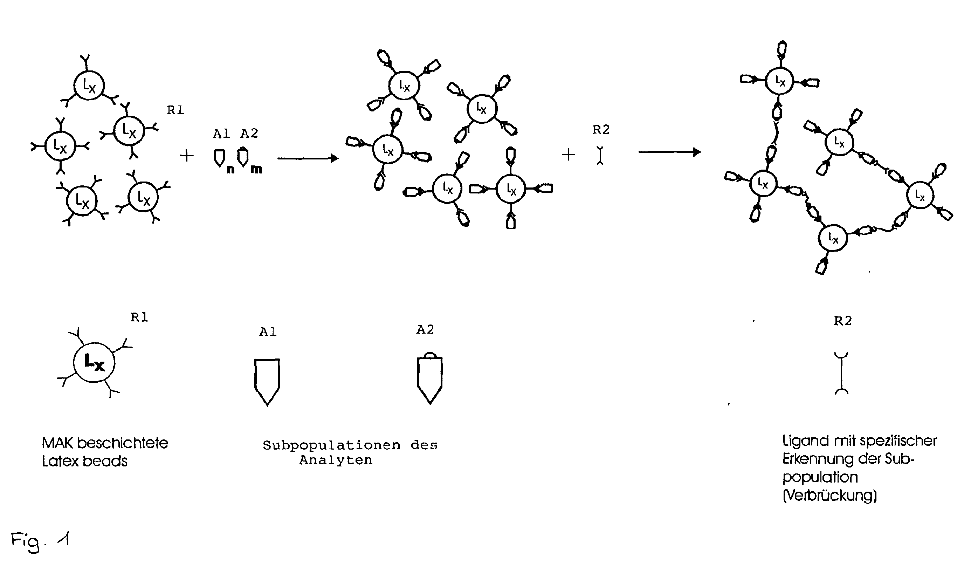

In Figur 1 der beiliegenden Zeichnung ist eine bevorzugte Ausführungsform des erfindungsgemäßen Verfahrens näher erläutert. Dabei werden beispielsweise mit monoklonalen Antikörpern R1 beschichtete Latexteilchen (Lₓ) mit einer Probe versetzt, die den zu bestimmenden Analyten enthält. Der zu bestimmende Analyt liegt als Gemisch von mindestens zwei Subpopulationen (A1 und A2), die in Anteilen von n bzw. m (100-n) % an der Gesamtmenge des Analyten vorhanden sind. Beide Subpopulationen des Analyten binden nun an die Latexpartikel. Eine Vernetzung findet nicht statt.

Im nächsten Reaktionsschritt wird ein zweiter löslicher Rezeptor R2 zugegeben, der spezifisch für die Subpopulation A2 des Analyten ist. Durch Zugabe von R2 findet eine Verbrückung der Latexpartikel statt, die zu einer Veränderung der optischen Eigenschaften des Meßmediums führt. Die Konzentration der Subpopulation A2 des Analyten kann durch die Geschwindigkeit oder/und das Ausmaß dieser Veränderung ermittelt werden.

Die Bestimmung des Analyten erfolgt vorzugsweise über die Messung der Streulichtintensität (Nephelometrie) oder über die Messung des Intensitätsverlusts von durch das Medium tretendem Licht (Turbidimetrie).

Die Konzentration des zu bestimmenden Analyten bzw. der zu bestimmenden Analytensubpopulation ist proportional zur Zunahme der optischen Absorption im Meßmedium. Zur Quantifizierung der erhaltenen Ergebnisse wird vorzugsweise eine Eichkurve erstellt. Hierzu werden vorzugsweise Proben, die bekannte Mengen des zu bestimmenden Analyten bzw. der zu bestimmenden Analytsubpopulation enthalten, vermessen. Der Gehalt des Analyten bzw. der Analytsubpopulation in einer zu bestimmenden unbekannten Probe wird nach Messung der Agglutinationsgeschwindigkeit aus der Eichkurve abgelesen.

Wenn der zu bestimmende Analyt ein Protein ist, kann man einen zweiten Rezeptor verwenden, der spezifisch für eine Subpopulation dieses Proteins ist, die sich durch Vorhandensein oder Abwesenheit einer Sekundärmodifikation von anderen Subpopulationen des Proteins unterscheidet. Solche Sekundärmodifikationen können etwa eine Glykosilierung, eine bestimmte Art von Glykosilierung, z.B. das Vorliegen einer bestimmten Kohlenhydratteilstruktur wie etwa eines Sialinsäurerests, eine Phosphorylierung oder eine Sulfatierung sein.

Ein Beispiel für Analytsubpopulationen, die durch das erfindungsgemäße Verfahren bestimmt werden können, sind bestimmte Glykosilierungsformen von Lipoproteinen, wie etwa die Asialoform von Apolipoprotein-B. Als erster Rezeptor kann hier beispielsweise ein Antikörper gegen das Lipoprotein verwendet werden, der für eine nur einmal vorkommende Oberflächenstruktur spezifisch ist, und als zweiter Rezeptor ein Lectin, z.B. das Lectin RCA 120 aus Ricinus communis. Analog kann zur Bestimmung von Sialo-Apolipoprotein B das Lectin SNA aus Sambucus nigra eingesetzt werden.

Weitere Beispiele für die Anwendung des erfindungsgemäßen Verfahrens sind der Nachweis von Proteinsubpopulationen mit Sekundärmodifikationen, wie z.B. Glykosilierung, Phosphorylierung, Sulfatierung, Vorhandensein von Lipidresten wie etwaFarnesyl- oder Myristoylresten, Vorhandensein von prosthetischen Gruppen etc. oder der Nachweis von unvollständig synthetisierten Proteinen. Dabei kann als erster Rezeptor ein für das jeweilige Protein spezifischer Antikörper und als zweiter Rezeptor ein Lectin, das gegen eine bestimmte Kohlenhydratgruppe gerichtet ist, oder ein weiterer Antikörper, der beispielsweise für Phosphotyrosin spezifisch ist, verwendet werden.

Eine spezifische Anwendung des erfindungsgemäßen Verfahrens ist der Nachweis von unglykosiliertem Transferrin in Seren von Alkoholikern. Hierbei wird als erster Rezeptor ein Antikörper, der eine nur ein einziges Mal vorkommende Oberflächenstruktur auf Transferrin erkennt verwendet. Zur Vernetzung, d.h. als zweiter Rezeptor, dient ein Antikörper, der eine normalerweise durch Glykosilierung verdeckte Oberflächenstruktur des Proteins bindet. Normal glykosiliertes Transferrin wird von diesem zweiten Rezeptor nicht erkannt, da die Glykosilierung die Bindung des Antikörpers verhindert.

Ein weiterer Gegenstand der vorliegenden Erfindung ist ein Reagenzienkit zur Durchführung des erfindungsgemäßen Verfahrens, umfassend
(a) einen einzigen ersten Rezeptor, der an ein erstes, nur einmal auf den zu bestimmenden Analyten vorhandenes Epitop bindet und auf einem teilchenförmigen Trägermaterial immobilisiert ist,
(b) ein zweiter Rezeptor, der an ein zweites Epitop auf dem Analyten bindet und mindestens zwei Bindungsstellen für das zweite Epitop aufweist, wobei das erste Epitop verschieden vom zweiten Epitop ist und
(c) gegebenenfalls geeignete Puffer.

Weiterhin soll die Erfindung durch die nachfolgenden Beispiele erläutert werden.

### BEISPIEL 1:

### Bestimmung von Asialo- und Sialo-Apolipoprotein B

### 1.1. Reagenzien

Zur Herstellung des ersten Rezeptors wurden 10 mg Carboxyaktivierte Latexpartikel mit 282 nm Durchmesser mit einem monoklonalen Maus-Anti-Apolipoprotein B-IgG (Boehringer Mannheim, Kat.Nr. 1484 273) nach einem Standardverfahren mit EDC (N-Ethyl-N'(3-dimethylaminopropyl)carbodiimid) gekoppelt. Die Latexsuspension wurde 20 min bei Raumtemperatur mit 10 mM Natriumperjodat behandelt, um die auf dem Antikörper vorhandenen Kohlenhydratgruppen zu oxidieren (vgl. Marshall und Neuberger "Structural analysis of the carbohydrate groups of glycoproteins: periodate Oxidation methods", in: Gottschalk, A.ed., Glycoproteins 2nd ed., Amsterdam, Elsevier, 1972, Part A, pp. 331-341) . Zur Inaktivierung der bei der Oxidation entstandenen Aldehydgruppen wurde die Suspension anschließend 5 min mit 1% Ethanolamin in 50 mM Trispuffer pH 9 behandelt. Nach der Behandlung wurde der Latex abzentrifugiert und in Lagerpuffer aufgenommen.

Als zweite Rezeptoren wurden die Lectine RCA 120 aus Ricinus communis (Boehringer Mannheim, Id.Nr. 0223719) und SNA aus Sambucus nigra (Vector, Kat.Nr. L-300) verwendet.

Gereinigtes Low Density Lipoprotein (LDL) wurde mittels Ultrazentrifugation nach der Methode von Lindgren et al. (Lipid 10 (1975), 750-756) hergestellt.

### 1.2. Testansatz und Messung

Unterschiedliche Mengen an Probe (1,25 - 10 µl LDL, gegebenenfalls Sialidase-behandelt) wurden zu 125 µl Puffer, 25 µl Latex (0,1 %) oder/und unterschiedlichen Mengen der Lectine RCA 120 bzw. SNA gegeben. Als Kontrollen wurden LDL (Apo B) freies Serum bzw. ein Serum mit bekanntem Apo B-Gehalt (Apo B kalibriertes Serum) verwendet. Die Inkubation erfolgte im Photometer bei 37°C. Die Messung erfolgte durch eine Verfolgung der Absorption bei 546 nm.

Die Ergebnisse dieses Experiments sind in der nachfolgenden Tabelle 1 dargestellt.

### 1.3. Auswertung der Ergebnisse

Tabelle 1 zeigt, daß durch das erfindungsgemäße Verfahren unter Verwendung der Rezeptorkombination Antikörper/Lectin RCA 120 eine Bestimmung der Asialo-Subpopulation von Apo B möglich ist. Bei Verwendung der Rezeptorkombination Antikörper/Lectin SNA gelingt eine Bestimmung der Sialo-Apolipoprotein B-Subpopulation.

Zur Quantifizierung der erhaltenen Ergebnisse wird eine Eichkurve erstellt. Hierzu wird eine LDL-Präparation durch Behandlung mit Sialidase vollständig desialyliert und eine weitere LDL-Probe wird durch Behandlung mit Sialyltransferase und CMP-NANA vollständig sialyliert. Aus diesen beiden Proben können Apolipoprotein B-Präparate hergestellt werden, deren Gehalt an Asialo-Apo B bekannt ist und die zur Erstellung der Eichkurve verwendet werden können. Der Gehalt an Asialo- oder Sialo-Apo B eines zu bestimmenden unbekannten Präparats wird nach Messung der Agglutinationsgeschwindigkeit aus der Eichkurve abgelesen.

**Tabelle 1**

| Ergebnisse 1. Serie Latexbeads | | | | | |
|---|---|---|---|---|---|
| | | | | | Abs. / min |
| | | | | | |
| Puffer + | Latex | | | | 0 |
| " | " | + 10 µL LDL | | | 0 |
| " | " | + RCA 120 (10 µg / mL) . | | | 0 |
| " | " | " | | + 1,25 µL LDL | 0.075 |
| " | " | " | | + 2,5 µL LDL | 0.14 |
| " | " | " | | + 5 µL LDL | 0.175 |
| " | " | " | | + 10 µL LDL | 0.19 |
| " | " | " | | + 10 µL ApoB Kalib. Serum | 0.09 |
| " | " | " | | + 10 µL ApoB freies Serum | 0 |
| | | | | | |
| Ergebnisse 2. Serie Latexbeads | | | | | |
| | | | | | |
| Puffer + | Latex | | | | 0 |
| " | " | + 10 µL LDL | | | 0 |
| " | " | + RCA 120 (1 0 µg / mL) | | | 0 |
| " | " | + RCA 120 (5 µg / mL) + 10 µL LDL | | | 0.04 |
| " | " | + RCA 120 (10 µg / mL) + 10 µL LDL | | | 0.075 |
| " | " | + RCA 120 (10 µg / mL) + 10 µL LDL Sialidase beh. | | | 0.1 |
| " | " | + RCA 120 (20 µg / mL) + 10 µL LDL | | | 0.09 |
| " | " | + RCA 120 (50 µg / mL) + 10 µL LDL | | | 0.19 |
| " | " | + RCA 120 (50 µg / mL) + 10 µL LDL Sialidase beh. | | | 0.33 |
| | | | | | |
| Ergebnisse 3. Serie Latexbeads | | | | | |
| | | | | | |
| Puffer + | Latex | + SNA (50 µg / mL) | | | 0 |
| " | " | " | + LDL freies Serum | | 0 |
| " | " | " | + 10 µL LDL | | 0.065 |
| " | " | " | + 10 µL LDL / Sialidase behandelt | | 0.01 |

## Patentansprüche

1. Verfahren zur Bestimmung eines Analyten in einer Probeflüssigkeit,
**dadurch gekennzeichnet,**
**daß** man die Probeflüssigkeit mit (a) einem einzigen ersten Rezeptor, der an ein erstes, nur einmal auf dem Analyten vorhandenes Epitop bindet und auf einem teilchenförmigen Trägermaterial immobilisiert ist, und (b) einem zweiten Rezeptor, der an ein zweites Epitop auf dem Analyten bindet und mindestens zwei Bindungsstellen für das zweite Epitop aufweist, inkubiert, wobei das erste Epitop verschieden vom zweiten Epitop ist, und den Analyten über die Bindung an beide Rezeptoren bestimmt.

2. Verfahren nach Anspruch 1,
**dadurch gekennzeichnet,**
**daß** das teilchenförmige Trägermaterial aus anorganischen Partikeln, Latexpartikeln, Liposomen und Zellen ausgewählt wird.

3. Verfahren nach Anspruch 1 oder 2,
**dadurch gekennzeichnet,**
**daß** man als Rezeptoren bindefähige Substanzen, ausgewählt aus der Gruppe, bestehend aus Antikörpern, Antikörperfragmenten und -derivaten, Lectinen, Aptameren und Membranrezeptoren, verwendet.

4. Verfahren nach Anspruch 3,
**dadurch gekennzeichnet,**
**daß** man als ersten Rezeptor monoklonale Antikörper, monospezifische polyklonale Antikörper oder/und Fragmente derartiger Antikörper verwendet.

5. Verfahren nach Anspruch 3,
**dadurch gekennzeichnet,**
**daß** man als ersten Rezeptor ein Lectin verwendet.

6. Verfahren nach Anspruch 4 oder 5,
**dadurch gekennzeichnet,**
**daß** man eine Antikörper/Lectin-Rezeptorkombination verwendet.

7. Verfahren nach Anspruch 6,
**dadurch gekennzeichnet,**
**daß** man einen Antikörper mit modifizierten Kohlenhydratgruppen verwendet, an die das Lectin nicht mehr binden kann.

8. Verfahren nach einem der vorgehenden Ansprüche,
**dadurch gekennzeichnet,**
**daß** die Bestimmung durch einen homogenen Assay erfolgt.

9. Verfahren nach einem der vorgehenden Ansprüche,
**dadurch gekennzeichnet,**
**daß** der Analyt als Gemisch von mindestens zwei Analytsubpopulationen vorliegt und daß man einen zweiten Rezeptor verwendet, der spezifisch für eine zu bestimmende Subpopulation des Analyten ist.

10. Verfahren nach Anspruch 9,
**dadurch gekennzeichnet,**
**daß** man den Anteil einer Subpopulation an der Gesamtmenge des Analyten bestimmt.

11. Verfahren nach Anspruch 9 oder 10,
**dadurch gekennzeichnet,**
**daß** der zu bestimmende Analyt ein Protein ist und daß man einen zweiten Rezeptor verwendet, der spezifisch für eine Subpopulation des Proteins ist, die sich durch Vorhandensein oder Abwesenheit einer Sekundärmodifikation von anderen Subpopulationen unterscheidet.

12. Verfahren nach Anspruch 11,
**dadurch gekennzeichnet,**
**daß** die Sekundärmodifikation eine Glykosilierung, eine bestimmte Art von Glykosilierung, eine Phosphorylierung eine Sulfatierung, das Vorhandensein von Lipidresten oder das Vorhandensein von prosthetischen Gruppen ist.

13. Reagenzienkit zur Bestimmung eines Analyten, umfassend
(a) einen einzigen ersten Rezeptor, der an ein erstes, nur einmal auf dem Analyten vorhandenes Epitop bindet und auf einem teilchenförmigen Trägermaterial immobilisiert ist,
(b) einen zweiten Rezeptor, der an ein zweites Epitop auf dem Analyten bindet und mindestens zwei Bindungsstellen für das zweite Epitop aufweist, wobei das erste Epitop verschieden vom zweiten Epitop ist, und
(c) gegebenenfalls geeignete Puffer.

## Claims

1. Method for the determination of an analyte in a sample liquid,
**characterized in that** the sample liquid is incubated with (a) a single first receptor which binds to a first epitope which is present only once on the analyte and is immobilized on a particulate carrier material and (b) a second receptor which binds to a second epitope on the analyte and has at least two binding sites for the second epitope, the first epitope being different from the second epitope, and the analyte is determined by means of the binding to both receptors.

2. Method as claimed in claim 1,
**characterized in that**
the particulate carrier material is selected from inorganic particles, latex particles, liposomes and cells.

3. Method as claimed in claim 1 or 2,
**characterized in that**
substances capable of binding selected from the group comprising antibodies, antibody fragments and antibody derivatives, lectins, aptamers and membrane receptors are used as receptors.

4. Method as claimed in claim 3,
**characterized in that**
monoclonal antibodies, monospecific polyclonal antibodies or/and fragments of such antibodies are used as the first receptor.

5. Method as claimed in claim 3,
**characterized in that**
a lectin is used as the first receptor.

6. Method as claimed in claim 4 or 5,
**characterized in that**
an antibody/lectin receptor combination is used.

7. Method as claimed in claim 6,
**characterized in that** an antibody with modified carbohydrate groups to which the lectin can no longer bind is used.

8. Method as claimed in one of the previous claims,
**characterized in that**
the determination is carried out by means of a homogeneous assay.

9. Method as claimed in one of the previous claims,
**characterized in that**
the analyte is present as a mixture of at least two analyte subpopulations and a second receptor is used which is specific for one subpopulation of the analyte to be determined.

10. Method as claimed in claim 9,
**characterized in that**
the proportion of a subpopulation to the total amount of the analyte is determined.

11. Method as claimed in claim 9 or 10,
**characterized in that**
the analyte to be determined is a protein and a second receptor is used which is specific for a subpopulation of the protein that differs from other subpopulations by the presence or absence of a secondary modification.

12. Method as claimed in claim 11,
**characterized in that**
the secondary modification is a glycosylation, a certain type of glycosylation, a phosphorylation, a sulphation, the presence of lipid residues or the presence of prosthetic groups.

13. Reagent kit for the determination of an analyte comprising
(a) a single first receptor which binds to an epitope which is only present once on the analyte to be determined and is immobilized on a particulate carrier material,
(b) a second receptor which binds to a second epitope on the analyte and has at least two binding sites for the second epitope, the first epitope being different from the second epitope and
(c) optionally suitable buffers.

## Revendications

1. Procédé de détermination d'un analyte dans un échantillon liquide, **caractérisé en ce que** l'on incube l'échantillon liquide avec (a) un premier récepteur seul qui se lie à un premier épitope, présent seulement une fois sur l'analyte et qui est immobilisé sur un matériau support particulaire, et (b) un deuxième récepteur qui se lie à un deuxième épitope sur l'analyte et présente au moins deux sites de liaison pour le deuxième épitope, dans lequel le premier épitope est différent du deuxième épitope, et on détermine l'analyte via la liaison aux deux récepteurs.

2. Procédé selon la revendication 1, **caractérisé en ce que** le matériau support particulaire est choisi parmi les particules inorganiques, les particules de latex, les liposomes et les cellules.

3. Procédé selon la revendication 1 ou 2, **caractérisé en ce que** l'on utilise comme récepteurs des substances capables de se lier, choisies dans le groupe constitué des anticorps, des fragments et dérivés d'anticorps, des lectines, des aptamères et des récepteurs membranaires.

4. Procédé selon la revendication 3, **caractérisé en ce que** l'on utilise comme premier récepteur des anticorps monoclonaux, des anticorps polyclonaux mono-spécifiques et/ou des fragments de tels anticorps.

5. Procédé selon la revendication 3, **caractérisé en ce que** l'on utilise comme premier récepteur une lectine.

6. Procédé selon la revendication 4 ou 5, **caractérisé en ce que** l'on utilise une combinaison de récepteurs anticorps/lectine.

7. Procédé selon la revendication 6, **caractérisé en ce que** l'on utilise un anticorps avec des groupes glucidiques modifiés, sur lesquels la lectine ne peut plus se lier.

8. Procédé selon l'une des revendications précédentes, **caractérisé en ce que** l'on effectue la détermination au moyen d'un essai homogène.

9. Procédé selon l'une des revendications précédentes, **caractérisé en ce que** l'analyte se présente sous la forme d'un mélange d'au moins deux sous-populations d'analytes et **en ce qu'**on utilise un deuxième récepteur, qui est spécifique d'une sous-population de l'analyte à déterminer.

10. Procédé selon la revendication 9, **caractérisé en ce que** l'on détermine la proportion d'une sous-population par rapport à la quantité totale de l'analyte.

11. Procédé selon la revendication 9 ou 10, **caractérisé en ce que** l'analyte à déterminer est une protéine et **en ce que** l'on utilise un deuxième récepteur, qui est spécifique d'une sous-population de la protéine, qui se différentie d'autres sous-populations par la présence ou l'absence d'une modification secondaire.

12. Procédé selon la revendication 11, **caractérisé en ce que** la modification secondaire est une glycosylation, un certain type de glycosylation, une phosphorylation, une sulfatation, la présence de restes lipidiques ou la présence de groupements prosthétiques.

13. Trousse de réactifs pour la détermination d'un analyte, comprenant
(a) un premier récepteur unique, qui se lie à un premier épitope présent seulement une fois sur l'analyte, et est immobilisé sur un matériau support particulaire,
(b) un deuxième récepteur, qui se lie à un deuxième épitope sur l'analyte et présente au moins deux sites de liaison pour le deuxième épitope, le premier épitope étant différent du deuxième épitope, et
(c) éventuellement un tampon approprié.
